# EUROPEAN PATENT SPECIFICATION

(11) **EP 2 792 371 B1**
(45) Veröffentlichungstag und Bekanntmachung des Hinweises auf die Patenterteilung: **31.08.2016**
(21) Anmeldenummer: 12846829.5
(22) Anmeldetag: 04.12.2012
(51) Int. Cl.: A61L 9/20, B60H 3/00

(54) **UV-LUFTDESINFEKTIONSVORRICHTUNG FÜR FAHRZEUGE**
UV AIR DISINFECTION DEVICE FOR VEHICLES
DISPOSITIF DE DESINFECTION D'AIR POUR VEHICULES

(30) Priorität: 12.12.2011 RU 2011150149
(43) Veröffentlichungstag der Anmeldung: 22.10.2014
(73) Patentinhaber: Kiknadze, Nikolai Dzhemalovich, 119019 Moscow (RU)
(72) Erfinder: KOUSTJUCHENKO, Sergei Vladimirovich, Dolgoprudny Moskovskaya obl. 141700 (RU); KIKNADZE, Nikolai Jemalovich, Moscow 119019 (RU); TIMAKOV, Sergei Vasilievich, Moscow 105484 (RU); SITNIKOV, Aleksandr Sergeevich, Moscow 105187 (RU); KARELIN, Aleksandr Aleksandrovich, Moscow 127422 (RU)
(74) Vertreter: Sloboshanin, Sergej
(86) Internationale Anmeldenummer: PCT/RU2012/001017
(87) Internationale Veröffentlichungsnummer: WO 2013/089589

(56) Entgegenhaltungen:
- EP-A1- 1 676 818
- EP-A1- 1 882 521
- WO-A1-2011/026619
- WO-A1-2011/057401

## Beschreibung

### Technisches Gebiet

Die vorgeschlagene Erfindung bezieht sich auf eine Anlage zur Luftdesinfizierung eines Fahrzeugs mit bakterizider UV-Strahlung gemäß dem Oberbegriff des Anspruchs 1 und kann in Be- und Entlüftungssystemen und Konditionierungssystemen für den Personentransport, vorzugsweise den Eisenbahntransport, verwendet werden.

### Vorheriger Stand der Technik

Bekannt aus der EP 1 676 818 ist ein System zur Filterung und Reinigung von Wasser, in dem im Stadium der Vorreinigung neben der Anwendung der Koagulierung und Filterung unter anderem die Desinfizierung des Wassers mit Hilfe von UV-Strahlung erfolgen kann. Dabei sind in Glasrohren angebrachte UV-Lampen in einem Magnetflockungsmittel enthaltenen Behältnis angeordnet, in dem ein Wasserstrom durch Rührblätter gerührt wird, und während des Fließens an den UV-Lampen vorbei durch deren Strahlung desinfiziert wird. Da die Flockungsmittelpartikel sowie andere Ausscheidungen an den Glasrohroberflächen kleben bleiben, verringert sich die Durchlässigkeit der UV-Strahlung. Die Beseitigung der Verschmutzungen von den Glasrohroberflächen erfolgt durch Ausspülung. Zu diesem Zweck werden die Glasrohre mit Hilfe von speziellen Hebevorrichtungen aus dem Wasser in die Luft gehoben und entweder chemisch oder mechanisch, z. B. durch Abstreifer, gereinigt.

Die WO 2011/026619 offenbart eine Anlage zur UV-Desinfizierung von Abwasser und Trinkwasser, umfassend mehrere UV-Lampen, die in Schutzrohren so eingeschlossen sind, dass jedes Rohr bezüglich der Längsachse der Lampe symmetrisch angeordnet ist. Die Anlage ist mit einem System zur Reinigung der Schutzrohre versehen. Das System besteht aus einem jedes Schutzrohr umgreifenden Reinigungsring, in dem ein Abstreifer angeordnet ist, der sich gegen die Schutzrohroberfläche stützt, sowie aus einem Elektroantrieb oder Hydraulikantrieb, welcher zur Bewegung von mit ihm verbundenen Ringen in Richtung des Längsachse der Lampe dient. Jeder Ring weist einen Rohransatz zur Zufuhr einer Reinigungsflüssigkeit auf, die unter Hochdruck von einer Druckquelle dem Abstreifer des Ringes in Richtung zum Schutzrohr zugeführt wird. Die mehreren Ringe sind mit einem gemeinsamen Halter versehen und werden durch ein gemeinsames Druckmittel angetrieben.

Bekannt aus der WO 2011/057401 ist ein System zur Reinigung von Lampenrohren von UV-Strahlungsquellen entlang ihrer Gesamtlänge, die in Wasserbehandlungsmodulsystemen verwendet werden. Die Vorrichtung besteht aus einem mindestens einen Teil der Lampenrohroberfläche umgreifenden und mit einem Schneideelement verbundenen Reinigungselement, welches aus einer Lage in die andere bewegbar ist, wobei bei dieser Bewegung das Reinigungselement die Verschmutzungen in den Peripherieteil des Lampenmoduls verschiebt, und ein Teil der Verschmutzungen durch das Schneideelement beseitigt wird. Beim Erreichen des Peripherieteils drückt das Reinigungselement auf die Verschmutzungen, während das Schneideelement die Verschmutzungen durchschneidet. Die von den Lampenrohren entfernten Verschmutzungen werden mit Wasserstrom weggespült. Die Reinigungs- und Schneideelemente sind an einem Schlitten befestigt, der mittels eines mechanischen Antriebs bewegt wird.

Die EP 1 882 521 offenbart eine Vorrichtung zur Wasserdesinfizierung, bestehend aus einem Gas-Flüssigkeitsmischer, in dem Wasser mit Ozon vermischt wird, aus einem Gas-Flüssigkeitsseparator mit mehreren in dem Gas-Flüssigkeitsseparator angeordneten UV-Lampen in Schutzhüllen zur Bestrahlung der gewonnenen Flüssigkeit, und aus einer Pumpe. Die Vorrichtung ist mit einer Einrichtung zur Reinigung der Lampenhüllen von Verschmutzungen versehen, wobei die Einrichtung Halter darstellt, in deren Öffnungen Abstreifer angeordnet sind. Die Halter werden entlang der Hüllen mittels eines durch einen Motor angetriebenen Antriebs bewegt.

Bekannt ist eine Anlage zur Luftdesinfizierung mit UV-Strahlung, unter anderem in Be- und Entlüftungssystemen und Konditionierungssystemen für den Eisenbahntransport, die ein Gehäuse darstellt, das eine Eingangsöffnung und eine Ausgangsöffnung mit einer innerhalb des Gehäuses angebrachten bakteriziden UV-Lampe aufweist. Der Aufbau der bekannten Vorrichtung gewährleistet ihren sicheren und effektiven Betrieb in Fahrzeugen mit hohem Vibrationsniveau (zum Beispiel in Eisenbahnwagen): die Ausführung der Verstärkungsrippen am Gehäuse der Anlage und die Befestigung der Lampe an den Vibrationsdämpfelementen erlaubt es, die Schwingungen, die auf die Lampe übertragen werden, erheblich zu vermindern oder zu dämpfen. Jedoch ist ein Nachteil der bekannten Vorrichtung das Fehlen eines Reinigungssystems zum Reinigen der Lampenoberfläche von Verschmutzungen, die ihren Strahlungswirkungsgrad beeinträchtigen. Die Notwendigkeit einer periodischen Reinigung der Lampe erschwert den Betrieb der genannten Anlage (Gebrauchsmuster der Russischen Föderation Nr. 78074, 01.07.2008, A 6L2/10).

Bekannt ist ein UV-Sterilisator für Luftkonditionierungssysteme an öffentlichen Orten, der aus einem offenen Gehäuse besteht, innerhalb welchem UV-Lampen angebracht sind, die mit einer Vorrichtung zur Reinigung der Oberfläche ausgestattet sind. Die Vorrichtung zur Reinigung besteht aus einem Halter, an dem ein ringförmiger Abstreifer befestigt ist, der die UV-Lampe umfasst, und aus einem mit dem Halter verbundenen Antrieb, mit dessen Hilfe die Abstreifer entlang der Lampe bewegt werden (chinesisches Patent Nr. 2722913, A 6L9/20, 2005). Ein Nachteil der bekannten Vorrichtung ist die Komplizierung des Aufbaus, die durch das Vorhandensein eines Antriebs zur Bewegung der Vorrichtung zur Reinigung entlang der Lampe und durch die Zufuhr der für diese erforderliche Energie bedingt ist.

### Offenbarung des Wesens der Erfindung

Der vorliegenden Erfindung liegt die Aufgabe zugrunde, eine im Betrieb einfachere und energiesparende Anlage zur Luftdesinfizierung unter Beibehaltung der Zuverlässigkeit und Effektivität bei ihrer Verwendung in Verkehrsmitteln zu entwickeln.

Das technische Ergebnis, das bei der Ausführung der vorgeschlagenen Erfindung erzielt wird, besteht in der Vereinfachung des Betriebs und des Aufbaus der Anlage zur Luftdesinfizierung von Fahrzeugen sowie in der Minderung des Energieaufwandes für ihre Bedienung.

Das angegebene technische Ergebnis wird dadurch erreicht, dass in der Anlage zur Luftdesinfizierung eines Fahrzeuges mit UV-Strahlung ein Gehäuse mit einer Eingangs- und einer Ausgangsöffnung vorhanden ist, wobei innerhalb des Gehäuses mindestens eine UV-Lampe und eine Vorrichtung zur Reinigung der Oberfläche der Lampe angebracht ist, wobei die Vorrichtung eine Führung entlang der Längsachse der Lampe enthält, entlang derer ein Bewegungselement bewegt wird, das mit Haltern verbunden ist, an denen Reinigungselemente (Abstreifer) angebracht sind, wobei gemäß der vorgeschlagenen technischen Lösung die Längsachse der Lampe und die Führung in die Richtung der Bewegung des Fahrzeugs orientierbar ist, und als Bewegungselement ein Gewicht verwendet wird, das in der Führung unter Einwirkung einer Treibkraft frei bewegbar gelagert ist, wobei die Treibkraft bei Änderung der Fahrgeschwindigkeit des Fahrzeuges entsteht..

### Kurze Beschreibung der Zeichnungen

Die Erfindung wird anhand einer Zeichnung erläutert, in der UV-Lampen 1 gezeigt sind, die innerhalb des Gehäuses der Anlage an Haltestangen 2 mit Hilfe von Befestigungen 3 angebracht sind, und eine Vorrichtung zur Reinigung der Lampenoberfläche, die eine Führung 4 enthält, die entlang der Längsachse der Lampe angeordnet ist, auf der ein frei bewegbares Gewicht 5 angebracht ist, das mit einem Halter 6 verbunden ist, wobei innerhalb des Halters ein ringförmiges Reinigungselement 7 angebracht ist, das die Lampenoberfläche umfasst.

### Ausführungsbeispiel

Die Anlage zur Luftdesinfizierung mit UV-Strahlung wird beispielsweise in den Rücklaufkanal eines Belüftungs- und Luftkonditionierungssystems eines Eisenbahnpersonenwagens eingebaut. Der Strom der mit Mikroorganismen infizierten rückläufigen Luft, dessen Richtung mit der Richtung der Bewegung des Zuges übereinstimmt, strömt aus dem Wagen durch die Eingangsöffnung im Gehäuse der Anlage in die Zone der UV-Bestrahlung ein, die von zwei bakteriziden Lampen gebildet wird, deren Längsachsen auch in die Richtung der Bewegung des Zuges orientiert sind. Die Lampen sind mit Hilfe von Vibrationsdämpfelementen befestigt, wobei in den Haltestangen Kolben angebracht sind, die in der Umbiegestelle in der Form eines Propellers gebogen sind, und in den Vibrationsdämpfungsbälgen Lampensockel angebracht sind. Als Quelle der bakteriziden UV-Strahlung werden zwei leistungsstarke ozonfreie Niederdruck-Gasentladungs-Amalgamlampen des Typs ANZ 215/95 in U-Form verwendet, die speziell für die Luftbehandlung bestimmt sind (Patent der Russischen Föderation Nr. 2325727). Unter Einwirkung der bakteriziden UV-Strahlung wird die Luft desinfiziert, über die Ausgangsöffnung entfernt und über einen Belüfter der Klimaanlage ins Wagenabteil abgegeben. Im Laufe der Luftdesinfizierung setzt sich auf die sich erwärmende Oberfläche der UV-Lampe ständig Staub ab, welcher einen Teil der bakteriziden Strahlung absorbiert, infolgedessen die Effektivität der Desinfizierung vermindert wird. Eine periodische Reinigung der Lampenoberfläche wird mit Hilfe einer Vorrichtung durchgeführt, die aus einer Führung entlang der Längsachse der Lampe besteht, an der ein Gewicht 5 frei bewegbar gelagert ist, welches mit einem Halter 6 verbunden ist, wobei innerhalb des Halters ein ringförmiges Reinigungselement 7 angebracht ist, das die Lampenoberfläche umfasst. Da die Längsachse der Lampe und die Führung in die Richtung der Bewegung des Zuges orientiert sind, bewegen sich die Gewichte in der Führung entlang der Lampe unter Einwirkung einer Treibkraft, die bei Änderung der Fahrgeschwindigkeit des Zuges (Bremsen, Beschleunigung) entsteht, frei hin oder zurück, wobei die Halter mit den in ihnen befestigten Abstreifern, welche Fluoroplastringe sind, mitgezogen werden. Indem die Abstreifer sich entlang der Lampe bewegen, beseitigen sie Verschmutzungen von der Lampenoberfläche.

Somit werden aufgrund der Nutzung der Treibkraft zum Antrieb des Reinigungssystems der Lampe eine zusätzliche Aufwendung von Energie und die Notwendigkeit eines speziellen Antriebs ausgeschlossen, was der wesentliche Vorteil der vorgeschlagenen Erfindung ist.

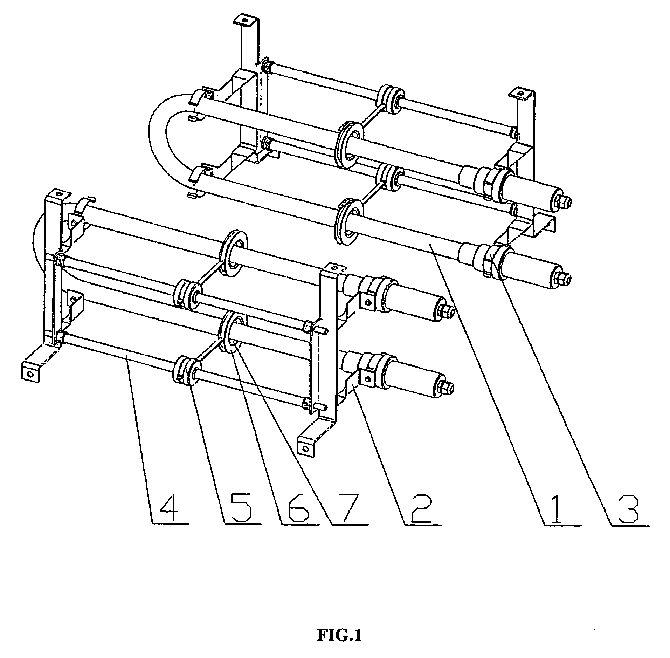

## Patentansprüche

1. Eine Anlage zur Luftdesinfizierung eines Fahrzeuges mit UV-Strahlung, wobei die Anlage aufweist: ein Gehäuse mit einem Eingang und einem Ausgang für den Luftstrom, wobei innerhalb des Gehäuses mindestens eine UV-Lampe (1) und eine Vorrichtung zur Reinigung der Oberfläche der Lampe (1) angebracht ist, wobei die Vorrichtung eine Führung (4) entlang der Längsachse der Lampe (1) enthält, an der ein Bewegungselement angebracht ist, das mit Haltern (6) verbunden ist, innerhalb derer Reinigungselemente (7) angebracht sind, **dadurch gekennzeichnet, dass** die Längsachse der Lampe (1) und die Führung (4) in der Richtung der Bewegung des Fahrzeugs orientiert sind, und als Bewegungselement ein Gewicht (5) verwendet wird, das in der Führung (4) unter Einwirkung einer Treibkraft frei bewegbar gelagert ist, wobei die Treibkraft bei Änderung der Fahrgeschwindigkeit des Fahrzeuges entsteht.

2. Die Anlage nach Anspruch 1, wobei das Reinigungselement (7) ringförmig gebildet ist und die Lampenoberfläche umfasst.

## Claims

1. An apparatus for disinfecting the air of a vehicle with UV-radiation, the apparatus comprising: a housing with an inlet and an outlet for the air flow, wherein within the housing at least a UV-lamp (1) and a device for cleaning the surface of the lamp (1) is mounted, said device comprising a guide (4) along the longitudinal axis of the lamp (1), wherein to said guide a motion element is attached, said motion element being connected with holders (6) within which cleaning elements (7) are mounted, **characterized in that** the longitudinal axis of the lamp (1) and the guide (4) are oriented in the direction of the movement of the vehicle, and as motion element a weight (5) is used which under the influence of a driving force is freely movably supported in the guide (4), wherein the driving force occurs upon change of the driving speed of the vehicle.

2. The apparatus according to claim 1, wherein the cleaning element (7) is annularly formed and comprises the surface of the lamp.

## Revendications

1. Une installation destinée à désinfecter l'air d'un véhicule par rayonnement UV, ladite installation comprenant: un boîtier doté d'une entrée et d'une sortie pour le flux d'air, à l'intérieur dudit boîtier étant montés au moins une lampe UV (1) et un dispositif de nettoyage de la surface de la lampe (1), ledit dispositif comportant un guide (4) agencé le long de l'axe longitudinal de la lampe (1) et sur lequel est monté un élément mobile qui est relié à des supports (6) à l'intérieur desquels sont montés des éléments de nettoyage (7), **caractérisé en ce que** l'axe longitudinal de la lampe (1) et le guide (4) sont orientables dans la direction de déplacement du véhicule, et comme élément mobile est utilisé un poids (5) monté dans le guide (4) de façon à se déplacer librement sous l'action d'une force motrice, ladite force motrice étant générée lorsque la vitesse de marche du véhicule change.

2. L'installation selon la revendication 1, l'élément de nettoyage (7) étant de forme annulaire et englobant la surface de la lampe.
